# EUROPEAN PATENT SPECIFICATION

(11) **EP 1 189 521 B1**
(45) Date of publication and mention of the grant of the patent: **25.10.2006**
(21) Application number: 00916123.3
(22) Date of filing: 06.03.2000
(51) Int. Cl.: A23L 1/30, A23L 1/275, A23L 3/3472, A61K 36/00

(54) **SOLUBLE PLANT DERIVED NATURAL COLOR CONCENTRATES AND ANTIMICROBIAL NUTRACEUTICALS**
KONZENTRATE NATÜRLICHER FARBSTOFFE SOWIE ANTIMIKROBIELLE NÄHRSTOFFE PFLANZLICHEN URSPRUNGS
CONCENTRES SOLUBLES DE COULEURS NATURELLES ET NUTRACEUTIQUES ANTIMICROBIENS A BASE DE PLANTES

(30) Priority: 05.03.1999 US 263046
(43) Date of publication of application: 27.03.2002
(73) Proprietor: Shanbrom Technologies, LLC, Ojai, CA 93023-3732 (US)
(72) Inventor: SHANBROM, Edward, Santa Ana, CA 92705 (US)
(74) Representative: Fiener, Josef
(86) International application number: PCT/US2000/005874
(87) International publication number: WO 2000/051445

(56) References cited:
- WO-A-93/13793
- WO-A-95/07623
- WO-A-95/26197
- WO-A-96/24341
- WO-A-97/29762
- WO-A-99/13889
- GB-A- 1 495 605
- GB-A- 2 036 535
- US-A- 3 431 208
- US-A- 4 588 589
- US-A- 5 108 763
- US-A- 5 989 560
- MITJAVILA S ET AL: "Tannin content of beverages. Separation and colorimetric titration of astringent polyphenolic fraction." ANNALES DE TECHNOLOGIE AGRICOLE 1971 INST. DE PHYSIOL., 84, GRANDE-RUE-SAINT-MICHEL, 31-TOULOUSE, FRANCE, vol. 20, no. 4, pages 335-346, XP000922738
- CANTARELLI C ET AL: "FORMALDEHYDE FOR PREVENTION OF OXIDATIVE BROWNING OF WHITE WINES" AMERICAN JOURNAL OF ENOLOGY AND VITICULTURE,XX,XX, vol. 22, no. 2, 1971, pages 59-64, XP000922739
- DATABASE WPI Section Ch, Week 199744 Derwent Publications Ltd., London, GB; Class A14, AN 1997-478208 XP002147075 & RU 2 075 965 C (CHUKHADZHYAN G A), 27 March 1997 (1997-03-27)
- DATABASE WPI Section Ch, Week 199431 Derwent Publications Ltd., London, GB; Class A96, AN 1994-253848 XP002147076 & RU 2 005 465 C (FITALON CO LTD), 15 January 1994 (1994-01-15)

## Description

### BACKGROUND OF THE INVENTION

### 1. Field of the Invention

The current invention concerns the field of natural products and foods and more specifically soluble colors and antimicrobial compositions prepared from cranberry and similar fruit juice.

### 2. Description of Related Art

Health foods are estimated to currently represent an annual market in the United States of at least ten billion dollars ($10,000,000,000.00). By "health foods" is meant vitamins, minerals and herbal products that are widely believed to be efficacious in improving human health without the cost and side effects of ordinary "artificial" pharmaceuticals. In recognition of the popularity and importance of these products the term "nutraceutical" or "functional foods" have been coined and the product category has received special government regulatory treatment.

There can be no denying that vitamins and minerals are essential for normal human health. Whether "excessive" doses of some vitamins, for example Vitamin C, provide special benefits is more controversial. More controversial still are the many herbal products of recent popularity such as saw palmetto, *Echinacea,* and *Ginkgo biloba.* It should be remembered that many important pharmaceutical drugs are based on natural plant products. For example, feverfew, long a folk cure for headaches, is currently used in many countries as a legitimate cure for migraines.

An even more widely known "natural cure" is the use of fruit juices, especially cranberry juice, for treatment and prevention of urinary tract infections. While the "cranberry juice cure" is widely prescribed, the precise basis of its effectiveness has been unknown. An early hypothesis was that the natural fruit acids, such as benzoic acid, acidified the urine and thereby inhibited bacterial proliferation. While acidification may be part of the puzzle, it does not seem sufficient to explain the advantage cranberry juice seems to hold over other acidic fruit juices. More recently there have been a number of reports that fruits of cranberry and related species of the genus *Vaccinium* contain potent factors that inhibit bacterial adhesion. Since bacterial must be able to adhere to urinary endothelia to cause an infection, the anti-adhesion factor may explain the cranberry effect. Some recent studies have identified the "anti-adhesion" factor with polyphenolic constituents of the juices-more particularly with anthocyanins and their precursors.

In fact, at least one research group has put extensive efforts into purification of the anti-adhesion factor from cranberry and related fruits. The reader's attention is drawn to a series of U.S. patents to Walker et al. (E.B. Walker, R.A. Mikelsen, J.N. Mikelsen and B.L. Roth) (including U.S. Patent Numbers 5,474,774, 5,525,341, and 5,646,178). These patents disclose complex extraction and fractionation processes by which cranberry fruits are extracted and yield a fraction enriched in the before-mentioned anti-adhesion factor. These patents provide tentative identification of the anti-adhesive factor. However, the Walker et al. process is complex and cumbersome. Further, it is not clear that all the benefits of cranberry and related fruits are due to the anti-adhesion factor.

In addition, there has been considerable recent research touting the benefits of "antioxidants" such as the polyphenolics such as the flavanoids or anthocyanins that are responsible for the color and purportedly the health benefits of red wine. Antioxidants or flavanoids are present in a large number of plants but there seems to be no simple or accepted means for purifying these components so they can be readily added to food or other products.

Therefore, there is still a need for a simple method to concentrate effective materials from cranberry and other plant sources (e.g., flowers, fruits, leaves, stems and roots) for nutraceutical and other uses. Besides their curative properties fruits and other plant materials are frequently strongly pigmented. Since much of our food is of plant origin people have become used to having foods with bright and appealing colors. Highly processed "artificial" foods are generally colorless or have drab and unappealing colors. Many millions of dollars each year are spent on putting "artificial colors" and "artificial flavors" into processed food products. While such additives may make the processed food products more attractive, they actually make the products even less suitable for human consumption. The worst of the carcinogenic coal tar dyes have been removed from the market, but a lingering doubt surrounds many of the remaining "certified food colors. " Thus, there is a significant need for methods to capture natural colors and flavors from fruits and vegetables.

### SUMMARY OF THE INVENTION

A soluble bioactive concentrate can be prepared from the juice (or aqueous homogenate) of cranberry and other fruits or vegetables by treating the juice with an appropriate binding material. The currently preferred material is soluble polyvinylpyrrolidone. The soluble binding material can be precipitated from solution by a number of manipulations such as decreasing the water activity (e.g., addition of hydrophilic solvents or solutes). The precipitated material is water-soluble in the absence of the additional solvents or solutes and shows significant antioxidant, antibacterial and antiviral properties. It can be readily consumed as a nutraceutical, it can be used topically, or it can be used as a safe food coloring. Significantly, the soluble binding material used in purification significantly stabilizes the colored materials. Whereas heat often destroys or damages natural plant pigments, the preparations of the present invention are stable to autoclaving and similar significant heating In addition, the soluble material is injectable into humans or animals so that it can be used directly as an injectable drug or as a preservative for injectable pharmaceuticals. The material is also useful as a preservative in foods, cosmetics, and drugs or biologicals. This same method is adapted to concentrating colors and flavors from a variety of fruits and vegetables.

### DETAILED DESCRIPTION

### OF THE PREFERRED EMBODIMENTS

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide a method for concentrating colors, biologically active fractions and flavors from fruits and vegetables (including flowers, leaves, stems, roots and "teas") and particularly antimicrobial and antiviral extracts from cranberry, blueberry, Aronia berry, grape and other fruit juices which method results in a substantially water-soluble product.

The present inventor has a long record of inventions in the medical field particularly in processes to disinfect blood and blood products. It was only natural that he would turn his inventive energies to the food industry where similar problems of dangerous pathogens exist. In the course of perfecting disinfecting purification methods for fruit juices the present inventor noticed that the iodine removal agents often removed some of the fruit juice color along with the iodine. This led to the question of whether these removal methods might be useful for concentrating fruit color or flavor or some other juice component. Experiments with a considerable number of different juices and binding agents then ensued. The resulting concentrated materials are potentially useful as color or flavor additives for food products. In addition, it has been discovered that some of the concentrates have unexpected antibacterial properties.

In earlier patent applications US 09/263,046 and US 08/931,315, the present inventor demonstrated that various insoluble binding materials, polyvinylpyrrolidone (PVP) and cholestyramine, in particular, are capable of extracting a colored polyphenolic fraction from fruit juices and other plant materials. Significantly the material extracted from fruit of plants of the genus *Vaccinium* and of the genus *Vitis* show strong antioxidant properties as well as unexpected antimicrobial and antiviral properties. As earlier disclosed materials have a multitude of uses. Both fruit juice and the binding agents used are considered safe for human consumption or for human skin and mucosa contact. The antimicrobials are especially useful in any treatments where bacterial growth is advantageously controlled. Such uses are in wound management where the material of the present invention can be inserted into bandages to prevent bacterial growth. It can also be directly applied to the wounds as part of a cleansing process. These novel antibacterials are also useful in treating periodontal disease where they can be used in place of antibiotics or traditional disinfectants such as peroxide. They can also be used in sanitary napkins and tampons to prevent the dangerous growth of *Staphylococcus,* which may result in Toxic Shock Syndrome.

Because the insoluble binding components are all of food grade and safe for human consumption, the insoluble juice factors are ideal as food coloring agents, preservatives, or as nutriceuticals. The components can also be bound to a suitable binding matrix such as PVP by a batch or single step removal process. It is also possible to apply a second binding matrix to the supernatant from the first binding to effect a "secondary capture" of additional components. The coloring components can be released (eluted) from the PVP or other binding matrix by changes in pH or ionic strength (e.g., buffers and salt solutions). However, it is still difficult to utilize the eluted or released materials. What is needed is a way to concentrate these materials into a solid form that can subsequently be dissolved in an aqueous solution. This would simplify the use of the concentrate either as a coloring or as a disinfectant material.

The present inventor has now perfected a method of producing such a concentrate. Juices or other plant-derived liquids are treated with water-soluble PVP or water-soluble polyvinyl alcohol (PVA), which have an affinity for the active components. Then the water-soluble PVP or PVA is "salted" out of solution through the addition of additional plant extract, additional PVP or PVA. Generally, soluble PVP, which has long been used to make "artificial" blood plasma, remains soluble even at very high concentrations. Adding more PVP to a simple aqueous solution merely results in a more viscous solution. However, when the polyphenolic plant factors are present, the behavior of PVP changes. It is believed that the polyphenolics crosslink the PVP molecules forming larger, more hydrophobic, structures. If additional polyphenolics are then added, these structures become insoluble. Addition of soluble PVP in the presence of the polyphenolics may also result in precipitation. Any of a number of actions that reduce the activity of water or "dehydrate" the solution will also cause the phenolic-PVP complex to precipitate. Besides actual removal of water activity can be altered through the addition of hydrophilic solvents or solutes. For example, various alcohols, glycols (polyethylene glycol, Pluronic surfactants, etc.), salts (e.g., sodium chloride, potassium chloride, calcium chloride, or other water soluble salts such as nitrates, sulfates, etc.), and hydrophilic solutes (e.g., amino acids, urea, and sugars) will cause precipitation of PVP-polyphenolic complex. It is believed that addition of any of these hydrophilic substances "draw" water away from the PVP-polyphenolic so that hydrophobic interactions predominate and the complex precipitates. Other means of dehydration can also be used, *e.g*., ultrafiltration, and evaporation.

In one experiment 40 ml of concentrated cranberry juice was added to 5.0 g of soluble PVP (MW = 30,000) and the mixture stirred until the PVP had dissolved. At this point 2 ml aliquots of the cranberry juice concentrate were added. with stirring between each addition. After 20 ml of concentrate had been added, formation of a precipitate was noted. The material was allowed to rest over night at room temperature. The solution was then centrifuged to concentrate the precipitate. Approximately 2 ml of a dark red precipitate was collected. The remaining supernatant was visibly lighter in color than the starting cranberry concentrate. One interesting observation is that the precipitate or a solution produced by dissolving the PVP-polyphenolic material in water is much more stable than the cranberry material alone. Normally the coloring material will readily photobleach or lose color from oxidation. The soluble PVP complex is much more light stable and resistant to oxidative breakdown. Further, the PVP complex is stable to prolonged autoclaving or similar heat treatment.

This technique appears to produce the most strongly colored PVP product. The extract can also be precipitated by adding aliquots of saturated PVP solution to the mixture or by adding aliquots of saturated "salt" solution. The "salt solution can be actual table salt (NaCl) or potassium or ammonium chloride. Other hydrophilic solutes such as urea also bring down the PVP-colored complex. In the preferred case of adding additional plant extract (e.g., juice) the precipitation is probably due to a crosslinking between adjacent PVP molecules which essentially converts the soluble PVP into insoluble (e.g., crosslinked) PVP. The important point is that the precipitated PVP complex remains water-soluble and can be readily dissolved in water or appropriate buffer.

To demonstrate effectiveness the precipitate and the supernatant remaining following precipitation were compared with a zone of inhibition test. In this experiment the precipitate showed a zone of inhibition two and one half times that of the supernatant. Further, since the fruit acids are present in the supernatant (but not in the precipitate), it is likely that much of the activity of the supernatant is due to acidity.

### Experiment 1

In this experiment the antiviral activity of soluble PVP prepared from cranberry juice and Aronia (fruit of the *Amelanchier* plant). The activity was compared to either a control or an equal weight of the crosslinked PVP extract of the same juices. For this experiment each of four 50 ml samples of whole blood was spiked one of four different viruses: VSV (vesicular stomatitis virus), EMC (equine myocarditis virus), BVD (bovine viral diarrhea) and PPV (porcine parvovirus). Each spiked sample was divided into five aliquots. To each aliquot one of the following samples was added (0.25 ml of 10% cranberry soluble PVP, 0.25 g of crosslinked cranberry PVP, 0.25 ml of 10% Aronia soluble PVP and 0.25 g of crosslinked Aronia PVP). The tubes were mixed and then allowed to incubate for 30 min at room temperature. At that point the samples were plated onto a VEPA (viral endpoint assay) as previously explained. Appropriate cell types were employed for each virus type. The VEPA assays were read and are shown in the following tables.

The 10% soluble PVP samples were prepared by precipitating soluble PVP as detailed above. Then a 10% aqueous solution of the colored PVP precipitate was prepared. In the experiments approximated equal weights (0.25 ml of aqueous solution versus 0.25 g of insoluble PVP) were used. Of course, the correspondence between the insoluble and the soluble PVP is harder to determine. It seems likely that the 10% PVP sample is considerably "more dilute" than the crosslinked PVP sample. If there is a rough correspondence between weight of PVP and weight of active plant extract bound, one should consider that the soluble PVP is only about 10% PVP (some proportion of that weight is actually plant extract) while the insoluble product is essentially 100% PVP (less whatever proportion of the material is plant extract). Thus, it is likely that the insoluble material is ten times more concentrated. However, the complete dissolution of the soluble material may enhance its measurable activity.

**Table 1.**

| VSV | | | | | | | | | | | | | | | | | |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | **Titer** |
| Soluble | 0 | 0 | 0 | 0 | 0 | 0 | 0 | 0 | 0 | 0 | 0 | 0 | 0 | 0 | 0 | 0 | **0** |
| cranberry | | | | | | | | | | | | | | | | | |
| XL | 0 | 0 | 0 | 0 | 0 | 0 | 0 | 0 | 0 | 0 | 0 | 0 | 0 | 0 | 0 | 0 | **0** |
| cranberry | | | | | | | | | | | | | | | | | |
| Soluble | 0 | 0 | 0 | 0 | 0 | 0 | 0 | 0 | 0 | 0 | 0 | 0 | 0 | 0 | 0 | 0 | **0** |
| Aronia | | | | | | | | | | | | | | | | | |
| XL | 0 | 0 | 0 | 0 | 0 | 0 | 0 | 0 | 0 | 0 | 0 | 0 | 0 | 0 | 0 | 0 | **0** |
| Aronia | | | | | | | | | | | | | | | | | |
| Control | 4 | 4 | 4 | 4 | 4 | 4 | 4 | 4 | 4 | 4 | 4 | 4 | 4 | 0 | 0 | 0 | **9.5** |

These results confirmed that VSV is very sensitive to both cranberry and Aronia extracts. The control represents PVP without plant extract. Additional titration experiments are necessary to compare the strength of the soluble to the crosslinked (XL) PVP.

**Table 2.**

| EMC | | | | | | | | | | | | | | | | | |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | **Titer** |
| Soluble cranberry | 0 | 0 | 0 | 0 | 0 | 0 | 0 | 0 | 0 | 0 | 0 | 0 | 0 | 0 | 0 | 0 | **0** |
| XL cranberry | 0 | 0 | 0 | 0 | 0 | 0 | 0 | 0 | 0 | 0 | 0 | 0 | 0 | 0 | 0 | 0 | **0** |
| Soluble Aronia | 0 | 0 | 0 | 0 | 0 | 0 | 0 | 0 | 0 | 0 | 0 | 0 | 0 | 0 | 0 | 0 | **0** |
| XL Aronia | 0 | 0 | 0 | 0 | 0 | 0 | 0 | 0 | 0 | 0 | 0 | 0 | 0 | 0 | 0 | 0 | **0** |
| Control | 4 | 4 | 4 | 4 | 4 | 4 | 4 | 2 | 0 | 0 | 0 | 0 | 0 | 0 | 0 | 0 | **5.6** |

These results indicate that EMC virus is also very susceptible to the cranberry and Aronia extracts.

**Table 3.**

| BVD | | | | | | | | | | | | | | | | | |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | **Titer** |
| Soluble cranberry | 4 | 4 | 4 | 4 | 4 | 4 | 0 | 0 | 0 | 0 | 0 | 0 | 0 | 0 | 0 | 0 | **4.6** |
| XL cranberry | 4 | 4 | 4 | 4 | 4 | 4 | 0 | 0 | 0 | 0 | 0 | 0 | 0 | 0 | 0 | 0 | **4.6** |
| Soluble Aronia | 4 | 4 | 4 | 4 | 4 | 4 | 0 | 0 | 0 | 0 | 0 | 0 | 0 | 0 | 0 | 0 | **4.6** |
| XL Aronia | 4 | 4 | 4 | 4 | 4 | 4 | 0 | 0 | 0 | 0 | 0 | 0 | 0 | 0 | 0 | 0 | **4.6** |
| Control | 4 | 4 | 4 | 4 | 4 | 4 | 0 | 0 | 0 | 0 | 0 | 0 | 0 | 0 | 0 | 0 | **4.6** |

These results indicate that the cranberry and Aronia extracts have no effect on BVD, at least at the concentrations used herein.

**Table 4.**

| PPV | | | | | | | | | | | | | | | | | |
|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | **Titer** |
| Soluble cranberry | 4 | 4 | 4 | 4 | 4 | 4 | 1 | 0 | 0 | 0 | 0 | 0 | 0 | 0 | 0 | 0 | **4.7** |
| XL cranberry | 4 | 4 | 4 | 4 | 4 | 4 | 0 | 0 | 0 | 0 | 0 | 0 | 0 | 0 | 0 | 0 | **4.6** |
| Soluble Aronia | 4 | 4 | 4 | 4 | 4 | 4 | 1 | 0 | 0 | 0 | 0 | 0 | 0 | 0 | 0 | 0 | **4.7** |
| XL Aronia | 4 | 4 | 4 | 4 | 4 | 4 | 2 | 0 | 0 | 0 | 0 | 0 | 0 | 0 | 0 | 0 | **4.9** |
| Control | 4 | 4 | 4 | 4 | 4 | 4 | 1 | 0 | 0 | 0 | 0 | 0 | 0 | 0 | 0 | 0 | **4.7** |

These results show that the presently used concentrations of cranberry and Aronia extract are ineffective against PPV although they may be effective at higher concentrations or with longer exposures.

In these experiments it should be kept in mind that while the concentration of effective agent delivered in the case of the crosslinked material is essential at a maximum, it is quite possible to significantly increase the concentration of the soluble PVP (as only a 10% dilution is presently used). These results tend to confirm the hypothesis that the pure soluble PVP material is about ten times as potent as the crosslinked product.

### Experiment 2

Further, with some plant materials the soluble versions appear to give vastly superior results as compared to the insoluble crosslinked material. This appears to be especially true with soluble PVP extract concentrated from the fruits of *Vitis* (grape), either *V. vinifera* (wine grape) or *V. labrusca* (Concord grape). Initial experiments with crosslinked PVP extracts showed relatively little antimicrobial activity. This is at least partly due to the fact that commercial Concord grape juice was the source of the test extracts. In the preparation of such juice a heat step is used to release the color from the fruit skins. Apparently, this heating decreases the favorable properties. When the extracts are prepared without heating, soluble grape PVP shows significant antibacterial activity against some fairly "difficult" bacteria. To a lesser degree this is also true of soluble PVP extracts prepared from fruit of *Rubus* (blackberry). Again, the lack of heat treatment prior to forming the PVP complex may be important.

Soluble grape-PVP and soluble blackberry-PVP were prepared as described above for cranberry-PVP. In addition, the materials were tested at either pH 5.0 or pH 7.0. For these tests aliquots of the test PVP extracts were adjusted to the desired pH and then sterile filtered using a 0.2µm filter. Serial two-fold dilutions were prepared using sterile typticase-soy broth. Each dilution was inoculated with 1x10⁴ test organisms per ml, and the solutions were incubated overnight at 35°C and then spread on agar growth plates. The readings in the following Table 5 indicate the highest serial dilution in which no organisms grew (*e.g*., the dilution at which the PVP extract killed or completely inhibited the bacteria

**Table 5**

| Test Organism | Grape (pH 5.0) | Grape (pH 7.0) | Blackberry (pH 5.0) | Blackberry (pH 7.0) |
|---|---|---|---|---|
| *Citrobacter freundii* | 1:128 | 1:2 | 1:8 | 1:4 |
| *Enterobacter cloacae* | 1:4 | 1:16 | n/a | 1:16 |
| *Enterobacter faecalis* | n/a | 0 | 1:4 | 0 |
| *Escherichia coli* | 1:16 | 0 | 1:8 | 0 |
| *Klebsiella pneumoniae* | 1:8 | 1:16 | 1:8 | 1:32 |
| *Pseudomonas aeruginosa* | 1:32 | 1:4 | 1:16 | 1:8 |
| *Salmonella enteritidis* | 1:16 | 0 | 1:16 | 1:8 |
| *Serratia marcescens* | 1:32 | 1:2 | 1:16 | 1:4 |
| *Staphylococcus aureus* | 1:2 | 1:8 | 1:4 | 1:16 |

It should be appreciated that the PVP extracts are killing or irreversibly inhibiting the bacteria in the presence of optimal growth conditions. In the presence of a less rich medium the extracts are even more effective. Also, if the bacteria are exposed to the extracts for longer time periods (several days) much higher dilutions (lower concentrations of the PVP extract) are able to completely inhibit the bacteria. This suggests that the PVP extracts somehow have a cumulative effect on the bacteria. Since there is some evidence that bacteria have a role in arterial disease, this may explain how a diet rich in plant polyphenolics shows a preventative effect on such disease.

### Experiment 3

This experiment was undertaken to test the hypothesis that resistant viruses like BVD would be susceptible to higher concentrations of the active extract and to make titration comparisons between the soluble PVP and the crosslinked PVP material. A tube containing 120 ml of fresh human blood was spiked with BVD and then divided into a plurality of 10 ml aliquots. A sample of either 10% soluble cranberry PVP (prepared as above) or crosslinked cranberry PVP was added to each tube. The tube was mixed and incubated for 30 min and then placed on a VEPA as before. As in the earlier experiments equivalent weights of the crosslinked and soluble PVP were used as is set out in Table 6.

**Table 6.**

| **10 % Soluble Cranberry-PVP** | **XL Cranberry-PVP** |
|---|---|
| Sample **a** = 500 µl | Sample **f** = 500 mg |
| Sample **b** = 750 µl | Sample **g** = 750 mg |
| Sample **c** = 1000 µl | Sample **h** = 1000 mg |
| Sample **d** = 1250 µl | Sample **i** = 1250 mg |
| Sample **e** = 1500 µl | Sample **j** = 1500 mg |

**Table 7.**

| BVD results with samples from Table 6. | | | | | | | | | | | | | |
|---|---|---|---|---|---|---|---|---|---|---|---|---|---|
| Samples | 1 | 2 | 3 | 4 | 5 | 6 | 7 | 8 | 9 | 10 | 11 | 12 | **Titer** |
| **a** | 4 | 4 | 4 | 4 | 4 | 1 | 0 | 0 | 0 | 0 | 0 | 0 | **4.7** |
| **b** | 4 | 4 | 4 | 0 | 0 | 0 | 0 | 0 | 0 | 0 | 0 | 0 | **4.6** |
| **c** | 0 | 0 | 0 | 0 | 0 | 0 | 0 | 0 | 0 | 0 | 0 | 0 | **0** |
| **d** | 0 | 0 | 0 | 0 | 0 | 0 | 0 | 0 | 0 | 0 | 0 | 0 | **0** |
| **e** | 0 | 0 | 0 | 0 | 0 | 0 | 0 | 0 | 0 | 0 | 0 | 0 | **0** |
| **f** | 4 | 4 | 4 | 4 | 0 | 0 | 0 | 0 | 0 | 0 | 0 | 0 | **3.1** |
| **g** | 4 | 4 | 4 | 0 | 0 | 0 | 0 | 0 | 0 | 0 | 0 | 0 | **2.7** |
| **h** | 0 | 0 | 0 | 0 | 0 | 0 | 0 | 0 | 0 | 0 | 0 | 0 | **0** |
| **i** | 0 | 0 | 0 | 0 | 0 | 0 | 0 | 0 | 0 | 0 | 0 | 0 | **0** |
| **j** | 0 | 0 | 0 | 0 | 0 | 0 | 0 | 0 | 0 | 0 | 0 | 0 | **0** |
| **Control** | 4 | 4 | 4 | 4 | 4 | 4 | 1 | 0 | 0 | 0 | 0 | 0 | **4.7** |

These results confirm the hypothesis that higher concentrations of the active agent is capable of killing a resistant virus such as BVD. It would appear that there is a fairly sharp concentration effect between the **b** and **c** or the **g** and **h** samples. This suggests that there is some sort of "site filling" effect. That is, the amount of active ingredient in 500 µl of soluble PVP extract is not adequate to show much effect, but double this concentration is fully effective. Presumably the first 500 µl worth is bound to the virus or used up in some ineffective manner. After those first sites are filled, then the active agent interacts with and destroys the virus. As a result, using more than enough material to saturate the first sites results in a significant increase in kill. These results also suggest that the crosslinked material is slightly more effective on a "equal" weight basis. Thus, the soluble material is not quite ten times as strong as the insoluble material (if it were fully ten times more effective, the crosslinked material would not appear to be slightly more effective). What is very significant is that concentrations of soluble PVP (e.g. sample c) that were effective at destroying the virus caused no apparent increase in hemolysis. Strong disinfecting agents (e.g., iodine) or detergents that are capable of destroying resistant viruses like BVD generally also damage cell membranes. This damage is apparent as increased hemolysis. This suggests that the soluble PVP-cranberry could be a safe antiviral agent for direct use in the blood stream or for inactivating virus in blood used for transfusion, fractionation, or laboratory testing.

### Experiment 4

Further experiments were undertaken to determine whether the active cranberry extracts were actually harmless to cells at concentrations that result in good virus kill. A number of prior experiments have demonstrated that two relatively easily measured blood parameters are indicative of damage to blood cells. When red cell membranes are damages, their ability to retain potassium (K⁺) is impaired. This is reflected in an increase of measured potassium in the plasma. Similarly, the enzyme LDH (lactate dehydrogenase) leaks from damaged cells so that higher levels of LDH reflect damage. Again, 10 ml tubes of fresh whole human blood were each treated with one of the additives shown in Table 8. After 30 min incubation at room temperature, the samples were observed for hemolysis (Table 9.), and LDH and potassium measurements were then made (Table 10.).

**Table 8.**

| **10 % Soluble Cranberry-PVP** | **XL Cranberry-PVP** |
|---|---|
| Sample **a** = 250 µl | Sample **g** = 250 mg |
| Sample **b** = 500 µl | Sample **h** = 500 mg |
| Sample **c** = 750 µl | Sample **i** = 750 mg |
| Sample **d** = 1000 µl | Sample **j** = 1000 mg |
| Sample **e** = 1250 µl | Sample **k** = 1250 mg |
| Sample **f** = 1500 µl | Sample **l** = 1500 mg |

**Table 9.**

| Hemolysis Determinations | |
|---|---|
| Soluble PVP Samples | Crosslinked PVP Samples |
| a) No Hemolysis | g) No Hemolysis |
| b) No Hemolysis | h) No Hemolysis |
| c) No Hemolysis | i) No Hemolysis |
| d) No Hemolysis | j) No Hemolysis |
| e) Slight Hemolysis | k) Slight Hemolysis |
| f) Slight Hemolysis | l) Slight Hemolysis |
| Control No Hemolysis | Control No Hemolysis |

**Table 10.**

| Chemistry Determinations | | | | | |
|---|---|---|---|---|---|
| **Sample** | **LDH** | **K**^{**+**} | **Sample** | **LDH** | **K**^{**+**} |
| **a** | 110 | 3.5 | g | 110 | 3.2 |
| **b** | 112 | 3.2 | **h** | 112 | 3.2 |
| **c** | 115 | 4.1 | **i** | 114. | 4.0 |
| **d** | 125 | 5.8 | **j** | 123 | 5.5 |
| **e** | 150 | 7.2 | **k** | 155 | 6.8 |
| **f** | 162 | 7.9 | **160** | 7.7 | |
| **control** | 110 | 3.2 | **control** | 110 | 3.2 |

These results indicate a close tracking between hemolysis and other indicia of cell damage. Significantly levels of extract (*e.g*. 1000 µl of soluble PVP extract), that are effective at destroying virus cause only slight cellular damage. It is likely that longer incubation with slightly lower concentrations of the active extract would result in total viral kill with no appreciable damage to the cells. One should keep in mind that soluble PVP has long been used in injectable liquids and is considered safe for injection purposes. The fact that the PVP-polyphenolic complex causes little cell damage indicates that it can be used as an injectable material. Either it can be added to other injectable drugs to preserve them and to kill any bacteria or virus (a great improvement over toxic mercury-containing preservatives such as thimerisol), or the material can be directly injected as an anti- bacterial or anti-viral drug.

### Experiment 5

An additional exciting property of the soluble PVP-polyphenolic complex should be mentioned. Not only is it inherently antimicrobial, it shows a synergistic effect with at least some other antimicrobial agents. The present inventor is just beginning to explore this effect but one example should be given. Lately it has been reasoned that many cases of stomach ulcers are actually the result of a bacterial infection *(Helicobacter pylori).* This bacterial is somewhat difficult to kill but a combination of traditional antibiotic drugs and bismuth (e.g. bismuth subsalicylate as in the common medicament PEPTO-BISMOL^{®}) has proven effective. The problem is that the long antibiotic treatment necessary often results in a serious disturbance in the patient's intestinal bacterial flora.

For this experiment the 10% PVP-polyphenolic complex was serially diluted by two-fold dilutions (e.g., 1:1, 1:2, 1:4, 1:8, etc.). Material from each dilution was spiked with bacteria and after a 30 minute incubation was plated on nutrient agar to check for bacterial growth. The titer of the polyphenolic complex was taken to be the most extreme dilution that still prevented growth of bacterial on the plates. The complex tested generally showed this point to be a dilution of 1:512. However, if 0.0005g of bismuth subsalicylate is added to each dilution, the titer improves to 1:1024 or better. However, this amount of bismuth has little if any effect on the bacteria by itself. Therefore, the polyphenolic complex shows a synergistic antibacterial effect with the bismuth. It is likely that this combination will prove useful in combating *H. pylori.* Additional experiments demonstrated that the synergistic effect works either with the soluble PVP or insoluble version of the antimicrobial factor.

The water-soluble character of the antimicrobial coloring composition of the present invention lends itself to uses in a variety of over the counter pharmaceutical products. The materials are particularly well suited as effective antimicrobials for mouthwashes or for use in lozenges for mouth cleaning and other functions. For such purposes the materials can be formulated as syrups, elixir, "spirits" or similar hydro-alcohol compositions. The formulae can be enhanced and sweetened with glycyrrhizins or related compounds, which also can show synergistic antimicrobial properties with the PVP-polyphenolic factors.

It should be appreciated that the present discovery of using soluble PVP to precipitate and concentrate polyphenolic and other biologically active compounds of plant origin is also useful with the previously described method of capturing such materials with crosslinked PVP, cholestyramine, crosslinked starch, and other insoluble binding materials. For example, cranberry-crosslinked PVP can be suspended and stirred in a concentrated solution of soluble PVP. Over a period of a few hours the vast majority of bound cranberry material transfers into the PVP solution, particularly if the liquid is heated to 40-50°C. Following the transfer, centrifugation or filtration removes the crosslinked PVP. Addition of more cranberry extract to the soluble PVP results in precipitation of soluble PVP-cranberry as described above. Alternatively, salting out the soluble PVP with other solutes can effect the precipitation. Although soluble PVP is the preferred complexing agent for use in the present invention, it is likely that other similar soluble organic polymers can also be used. For example, good results have also been obtained by replacing PVP with soluble polyvinyl alcohol.

It is clear that the present invention of soluble factors extracted from fruit juices or other plant products have a wide range of applications. Their soluble nature lends them to the preservation and disinfection of blood, blood products, pharmaceuticals and foods. They may also be useable as injectable drugs because they cause little or no cellular damage. These materials can also be used in most applications where the insoluble plant extract materials are useful. In addition, they are especially useful in coloring food and other products because of their soluble nature.

## Claims

1. A method for producing water-soluble germicidal factors comprising the steps of:
(a) contacting fruit juice or aqueous plant material homogenate with water-soluble polyvinylpyrrolidone or water-soluble polyvinyl alcohol:
(b) precipitating the germicidal factors by adding additional fruit juice or plant material homogenate; and
(c) harvesting the precipitated germicidal factor, wherein the precipitated factors may be readily redissolved In water.

2. The method of Claim 1, wherein the fruit juice or plant material homogenate is selected from the group consisting of cranberry juice, blueberry juice, blackberry juice, grape Juice and Aronia berry juice.

3. The method of Claim 1, wherein the fruit juice or plant material homogenate is produced from fruit of a species of the genus *Vaccinium.*

4. The method of Claim 1, wherein precipitating the germicidal factor is further potentiated by dehydration or by addition of a hydrophilic solute.

5. The method of Claim 4, wherein said additional hydrophilic solute is selected from the group consisting of water-soluble polyvinylpyrrolidone, water-soluble polyvinyl alcohol, urea, potassium chloride, sodium chloride, and calcium chloride.

6. The method of Claim 1, wherein step (a) is replaced by the steps of treating the fruit juice or plant material homogenate with insoluble polyvinyl pyrollidone or cholestyramine to render the water-soluble germicidal factors insoluble, and contacting the insoluble germicidal factors with an aqueous solution of soluble polyvinyl pyrollidone or polyvinyl alcohol to redissolve the germicidal factors.

7. Use of the water-soluble germicidal factors of claim 1 for the production of an antimicrobial tampon produced by placing the water-soluble germicidal factors of Claim 1 within a tampon.

8. Use of the water-soluble germicidal factors of Claim 1 combined with an antimicrobially effective amount of a bismuth salt for the manufacture of a medicament for treating digestive tract bacterial infections.

9. Use of the water-soluble germicidal factors of claim 1 for protecting a food product from bacterial contamination by adding the water-soluble germicidal factors of Claim 1 to prevent bacterial growth in the food product.

10. A method of producing a water-soluble coloring factors from flowers, fruits or vegetables comprising the steps of:
treating the flowers, fruits or vegetables to make an aqueous liquid homogenate;
contacting the aqueous liquid homogenate with water-soluble polyvinylpyrrolidone or water-soluble polyvinyl alcohol;
precipitating the water-soluble coloring factors by adding more water-soluble polyvinylpyrrolidone or water-soluble polyvinyl alcohol; and
harvesting the precipitated coloring factors, wherein the precipitated factors may be readily redissolved in water.

## Patentansprüche

1. Verfahren zur Herstellung wasserlöslicher, keimtötender Faktoren, umfassend die Schritte:
(a) Kontaktierung von Fruchtsaft oder wässrigem Pflanzenmaterial-Homogenat mit wasserlöslichem Polyvinylpyrrolidon oder wasserlöslichem Polyvinylalkohol:
(b) Präzipitation der keimtötenden Faktoren durch Zugabe von zusätzlichem Fruchtsaft oder Pflanzenmaterial-Homogenat; und
(c) Ernte der präzipitierten, keimtötenden Faktors, wobei die präzipitierten Faktoren leicht in Wasser rückgelöst werden können.

2. Verfahren nach Anspruch 1, worin der Fruchtsaft oder das Pflanzenmaterial-Homogenat aus der aus Kulturpreiselbeer-Saft, Blaubeer-Saft, Brombeer-Saft, Trauben-Saft und Aroniabeer-Saft bestehenden Gruppe ausgewählt ist.

3. Verfahren nach Anspruch 1, worin der Fruchtsaft oder das Pflanzenmaterial-Homogenat aus Früchten einer Art aus der Gattung *Vaccinium* hergestellt ist.

4. Verfahren nach Anspruch 1, worin die Präzipitation des keimtötenden Faktors durch Dehydrierung oder durch Zugabe eines hydrophilen, gelösten Stoffes weiter verstärkt wird.

5. Verfahren nach Anspruch 4, worin der zusätzliche hydrophile, gelöste Stoff aus der aus wasserlöslichem Polyvinylpyrrolidon, wasserlöslichem Polyvinylalkohol, Harnstoff, Stickstoff, Kalium, Chlorid, Natriumchlorid und Calciumchlorid bestehenden Gruppe ausgewählt ist.

6. Verfahren nach Anspruch 1, worin Schritt (a) durch die Schritte ersetzt wird: Behandeln des Fruchtsaftes oder Pflanzenmaterial-Homogenats mit unlöslichem Polyvinylpyrrolidon oder Cholestyramin, um die wasserlöslichen, keimtötenden Faktoren unlöslich zu machen; und Kontaktieren der unlöslichen, keimtötenden Faktoren mit einer wässrigen Lösung von löslichem Polyvinylpyrrolidon oder Polyvinylalkohol, um die keimtötenden Faktoren rückzulösen.

7. Verwendung der wasserlöslichen, keimtötenden Faktoren nach Anspruch 1 zur Herstellung eines antimikrobiellen Tampons, hergestellt durch Platzierung der wasserlöslichen, keimtötenden Faktoren nach Anspruch 1 in einem Tampon.

8. Verwendung der wasserlöslichen, keimtötenden Faktoren nach Anspruch 1 in Verbindung mit einer antimikrobiell wirksamen Menge an Bismut-Salz zur Herstellung eines Medikaments zur Behandlung bakterieller Infektionen des Verdauungstraktes.

9. Verwendung der wasserlöslichen, keimtötenden Faktoren nach Anspruch 1 zum Schutz eines Nahrungsmittelproduktes vor bakterieller Kontamination durch Zugabe der wasserlöslichen, keimtötenden Faktoren nach Anspruch 1, um bakterielles Wachstum im Nahrungsmittelprodukt zu verhindern.

10. Verfahren zur Herstellung wasserlöslicher Färbefaktoren aus Blüten, Früchten oder Gemüse, umfassend die Schritte:
Behandeln der Blüten, Früchten oder Gemüse, um ein wässriges, flüssiges Homogenat herzustellen; Kontaktieren des wässrigen, flüssigen Homogenats mit wasserlöslichem Polyvinylpyrrolidon oder wasserlöslichem Polyvinylalkohol; Präzipitieren der wasserlöslichen Färbefaktoren durch Zugabe von mehr löslichem Polyvinylpyrrolidon oder wasserlöslichem Polyvinylalkohol; und Ernten der präzipitierten Färbefaktoren, wobei die präzipitierten Faktoren leicht in Wasser rückgelöst werden können.

## Revendications

1. Procédé pour produire des facteurs germicides hydrosolubles comprenant les étapes de :
(a) mise en contact de jus de fruits ou d'homogénat de produit végétal aqueux avec de la polyvinylpyrrolidone hydrosoluble ou du poly(alcool vinylique) hydrosoluble ;
(b) précipitation des facteurs germicides par addition de jus de fruits ou d'homogénat de produit végétal supplémentaire ; et
(c) récolte du facteur germicide précipité, où les facteurs précipités peuvent être redissous aisément dans l'eau.

2. Procédé selon la revendication 1 où le jus de fruits ou l'homogénat de produit végétal est choisi dans le groupe consistant en le jus d'airelles, le jus de myrtilles, le jus de mûres, le jus de raisin et le jus de baies d'Aronia.

3. Procédé selon la revendication 1 où le jus de fruits ou l'homogénat de produit végétal est produit à partir de fruits d'une espèce du genre *Vaccinium.*

4. Procédé selon la revendication 1 où la précipitation du facteur germicide est renforcée encore par déshydratation ou par addition d'un soluté hydrophile.

5. Procédé selon la revendication 4 où ledit soluté hydrophile supplémentaire est choisi dans le groupe consistant en la polyvinylpyrrolidone hydrosoluble, le poly(alcool vinylique) hydrosoluble, l'urée, le chlorure de potassium, le chlorure de sodium et le chlorure de calcium.

6. Procédé selon la revendication 1 où l'étape (a) est remplacée par les étapes de traitement du jus de fruits ou de l'homogénat de produit végétal avec de la polyvinylpyrrolidone ou de la cholestyramine insoluble pour rendre insolubles les facteurs germicides hydrosolubles, et de mise en contact des facteurs germicides insolubles avec une solution aqueuse de polyvinylpyrrolidone ou de poly(alcool vinylique) soluble pour redissoudre les facteurs germicides.

7. Utilisation des facteurs germicides hydrosolubles selon la revendication 1 pour la production d'un tampon antimicrobien produit par mise en place des facteurs germicides hydrosolubles selon la revendication 1 dans un tampon.

8. Utilisation des facteurs germicides hydrosolubles selon la revendication 1 combinés avec une quantité efficace du point de vue antimicrobien d'un sel de bismuth pour la fabrication d'un médicament pour traiter les infections bactériennes des voies digestives.

9. Utilisation des facteurs germicides hydrosolubles selon la revendication 1 pour protéger un produit alimentaire contre une contamination bactérienne par addition des facteurs germicides hydrosolubles selon la navigation 1 pour empêcher la croissance bactérienne dans le produit alimentaire.

10. Procédé de production de facteurs colorants hydrosolubles à partir de fleurs, de fruits ou de légumes comprenant les étapes de :
traitement des fleurs, des fruits ou des légumes pour produire un homogénat liquide aqueux ;
mise en contact de l'homogénat liquide aqueux avec de la polyvinylpyrrolidone ou du poly(alcool vinylique) hydrosoluble ;
précipitation des facteurs colorants hydrosolubles par addition d'un supplément de polyvinylpyrrolidone ou de poly(alcool vinylique) hydrosoluble ; et
récolte des facteurs colorants précipités, où les facteurs précipités peuvent être aisément redissous dans l'eau.
